Europäisches Patentamt

European Patent Office

Office européen des brevets

(11) Publication number: **0 335 451**
**A2**

(12) **EUROPEAN PATENT APPLICATION**

(21) Application number: 89200734.5

(22) Date of filing: 22.03.89

(51) Int. Cl.4: **C12N 5/00** , **A01H 1/00** ,
**C12N 15/00** , **C12N 1/20**

(30) Priority: 25.03.88 NL 8800756

(43) Date of publication of application:
04.10.89 Bulletin 89/40

(84) Designated Contracting States:
AT BE CH DE ES FR GB GR IT LI LU NL SE

(71) Applicant: **VERENIGING VOOR CHRISTELIJK WETENSCHAPPELIJK ONDERWIJS**
De Boelelaan 1105
NL-1081 HV Amsterdam(NL)

(72) Inventor: **van der Krol, Alexander Ronald**
**Tademastraat 11**
**NL-1061 VK Amsterdam(NL)**
Inventor: **Stuitje, Antoine Raymond**
**Meidoornweg 30**
**NL-1171 JW Badhoevedorp(NL)**
Inventor: **Gerats, Antonius Gerardus**
**Bleekerslaan 3**
**NL-1814 EJ Alkmaar(NL)**
Inventor: **Mol, Josephus Nicolaas Maria**
**Fazantstraat 6**
**NL-1171 HS Badhoevedorp(NL)**

(74) Representative: **Smulders, Theodorus A.H.J.,**
**Ir. et al**
**Vereenigde Octrooibureaux Nieuwe Parklaan**
**107**
**NL-2587 BP 's-Gravenhage(NL)**

(54) Genetically engineered plant cells and plants, as well as recombinant DNA suitable therefor.

(57) The invention relates to genetically engineered plant cells which have been provided with an antisense gene of a natural gene of the host. Preferably, the gene concerned is a gene of the phenyl propanoid pathway, in particular the gene coding for chalcone synthase (CHS). Introduction of antisense CHS cDNA in Petunia results in different flower pigmentation phenotypes. The invention extends to recombinant DNA comprising the antisense gene.

FIG-3

# Genetically engineered plant cells and plants, as well as recombinant DNA suitable therefor.

## Field of the invention

The present invention relates to the field of recombinant DNA technology and more in particular to the field of genetic engineering of plant cells and plants using recombinant DNA.

## Background of the invention

### 1. Introduction

Both in prokaryotes and in eukaryotes gene expression can be controlled by products of regulator genes. It has been found that in specific cases, besides protein products encoded by these regulator genes, (untranslated) transcripts encoded by regulator genes can also influence the gene expression. The regulator RNA contains a nucleotide sequence complementary to a specific mRNA. Binding of the two transcripts occurs by base pairing. Both in vitro and in vivo, translation of the mRNA no longer occurs after hybridization of the mRNA with a complementary RNA.
The generally accepted molecular mechanism of this inhibition is the formation of an mRNA: complementary RNA hybrid. In prokaryotes the formation of such a double-stranded RNA hybrid leads to inhibition of the translation; in eukaryotes to inhibition of the RNA processing, inhibition of the RNA transport from the cell nucleus to the cytoplasm, blocking of the translation in the cytoplasm, a changed rate of RNA turnover, or a combination of these effects. Besides by the term antisense RNA, these regulator transcripts are also designated by the terms anti mRNA, complementary RNA, nonsense RNA or mic RNA (mRNA interfering complementary RNA). The genes coding for antisense RNA are called antisense genes.

The antisense RNA mechanism was discovered in prokaryotes. Bacteria use this regulator system, inter alia, in controlling the DNA replication and incompatibility of plasmids, such as ColE 1, CloDF 13, PSF 1030, p 15A, R1, NR 1, R 6-5 and pT 181, in the regulation of the Tn-10 transposition, in the osmoregulation in E. coli and in the regulation of phage reproduction. Although there are indications that in eukaryotes, too, antisense RNA is used as a regulator mechanism, this has not yet been proved.

The discovery that antisense RNA can inhibit the gene expression in natural systems, led to a number of experiments in which endeavours were made to artificially regulate genes by means of this technique. Manipulation of the gene expression via experimentally administered antisense RNA proved to be possible both in prokaryotes and in eukaryotes. Blocking or reducing the protein synthesis with antisense RNA proves to function in many cell types, including bacteria, cultivated mammalian cells, Xenopus and Drosophila embryos, Dictyostelium and plant cells. With relatively simple manipulations, antisense RNA complementary to a chosen mRNA can be synthesized in vivo or in vitro. Synthesis of antisense RNA may occur if excision of the sequence of a cloned gene is followed by insertion of the same fragment in reverse orientation relative to the promoter.

For induction of antisense RNA in prokaryotic and/or eukaryotic cells two methods are followed:

1. In vitro synthesis of oligonucleotides or antisense RNAs which are complementary to a specific mRNA, and which are then introduced into the cell.

2. Introduction into the cell of antisense genes. If the antisense gene is provided with a functional promoter, the cellular transcription system provides for the in vivo production of the desired antisense RNA. The promoter may be a constitutive promoter or a controllable or inducible promoter.

By means of microinjection large amounts of in vitro synthesized antisense RNA or complementary oligonucleotides can be directly introduced into the cell. Because of the limited life of the complementary transcripts the effects obtained are only temporary in nature ("transient expression"). Integration of an antisense gene into the genome, or stable maintenance of an antisense gene on a plasmid leads to a continuous antisense RNA production.

When determining the cellular functions of a gene it is often informative if the synthesis or the activity of the gene product is inhibited in full or in part. Identification and characterization of genes and their gene products, however, often proceed with difficulty, because mutations in such genes are often lethal, or (in polyploid organisms) recessive. Transformation techniques, however, often render it possible to add an additional, if required mutated, gene to the genome, but the original gene also remains present. Antibiotics or antibodies can be introduced into living cells as specific inhibitors of a gene product. This, however, requires characterization of the target protein and of the inhibitor. Selective inhibition of gene expression with antisense RNA would be an acceptable addition to the prevailing techniques; specific genes can be switched "on and off" or modulated (partial inhibition) without it being necessary to fully characterize the gene product. The antisense RNA is a new aid in the identification of cloned genes. Antisense RNA may also be used to prevent the production of phage, viral and oncogenetic proteins in vivo.

## 2. Antisense RNA in prokaryotes

In prokaryotes it has been found that inhibition of gene functions by antisense RNA is a natural process.

The regulatory role of small complementary RNA molecules was shown for the first time in the examination of plasmid replication in E. coli. The replication and the incompatibility property of the small E. coli plasmid ColE 1 and of the related plasmids CloDF 13, pBR 322, PSF 1030, p 15A is controlled by the interaction of two transcripts (complementary to each other): RNA I and RNA II. The RNA II forms a hybrid with the template DNA in the replication origin. Then this hybridized RNA II is digested to form the primer for the DNA synthesis of the plasmid. Transcription of RNA I occurs in a region which also codes for RNA II, but in the reverse direction. Binding of this RNA I to RNA II inhibits the hybridisation between the RNA II and the template DNA, so that primer formation is no longer possible; no replication of the plasmid takes place.

The replication and incompatibility of E. coli IncF II plasmids R1, NR 1 and R 6-5 which are not related to ColE 1 is likewise regulated via an RNA: RNA interaction. The gene product of gene repA is essential to plasmid replication. The natural antisense RNA (90 nucleotides in length) is complementary to the 5' end of the repA mRNA. Hybridisation of the antisense RNA with the repA mRNA inhibits the translation of the repA mRNA, so that plasmid replication cannot occur. Accordingly, contrary to ColE 1 and related plasmids not the primer formation but the repA translation is regulated via this antisense RNA mechanism.

The replication of the 4.4 kb Staphylococcus aureus plasmid pT 181 takes place in nearly the same manner as described for the E. coli IncF II plasmids. However, two natural antisense transcripts are involved here.

Although both with pT 181 and with the ColE 1 and IncF II group the replication of the plasmids is regulated with an antisense mechanism, there is no question of one general antisense transcript used with these three plasmid groups. The fact is that there is no significant base sequence homology between the antisense transcripts of pT 181 and those of the ColE 1 and IncF II group.

Another example of antisense control of bacterial translation is the regulation of the production of Tn-10 transposase in E. coli. Here a double-stranded RNA hybrid is formed between a small antisense RNA being 180 nucleotides in length and the Tn-10 transposase mRNA. No translation of the transposase mRNA can take place by hybridization of the antisense RNA with the transposase mRNA. Since the transposition frequency in vivo depends on the transposase level in the cell, the Tn-10 transposition frequency can thus be regulated.

Although the regulatory effect of RNA molecules in plasmid replication and in the production of Tn-10 transposase was known, the significance, the (possible) universality and the uses of the RNA inhibition technique were recognized only later, namely when the natural antisense ompF RNA was discovered. The expression of the E. coli genes for the outer membrane proteins ompC and ompF depends on the osmolarity of the culture medium. These outer membrane proteins function as diffusion pores for small hydrophilic molecules. The total amount of these proteins remains constant, but the mutual ratio depends on the medium osmolarity. In case of high osmolarity the outer membrane comprises mainly ompC and very little ompF. The fact is that in case of high osmolarity bidirectional transcription of ompC takes place. The thus obtained natural antisense transcript being 174 nucleotides in length is complementary to the translation start region of the ompF mRNA and inhibits the production of the ompF protein.

For the translation blockage of gene 1.1 in the bacteriophage T 7 mutant an antisense regulator system is likewise assumed, as in the regulation of the synthesis of the E. coli cAMP receptor protein. Antisense RNA is also one of the regulator factors in the phage lambda development. The synthesis of both the Q protein and the OPP RNA is probably controlled by natural antisense transcripts. Synthesis of the anti repressor protein of the Salmonella phage p 22 is likewise negatively regulated by means of a natural antisense transcript.

The antisense inhibition mechanism can also be applied experimentally in order to control the expression of genes endogenously present in the organism and/or experimentally introduced into the cells. For this purpose synthetic oligonucleotides or in vitro synthesized antisense RNA can be introduced into the cells. Further, antisense genes can be introduced into the cells which will then produce in vivo antisense RNA.

Synthetic complementary oligonucleotides can inhibit the translation in prokaryotes by hybridization to mRNAs, both in vitro and in vivo. Gene 32 of bacteriophage T 4 codes for a protein which plays a very important role in the phage DNA replication, recombination and repair. In vitro regulation of the gene 32 protein synthesis proved to be possible by means of 10- and 15-mer synthetic oligonucleotides complementary to the ribosome binding site of the gene 32 mRNA (Toulmé et al., 1986, PNAS 83, 1227-1231).

Because of the limited life of transcripts the effects obtained after microinjection into the cells of in vitro synthesized antisense RNA or complementary oligonucleotides are only temporary in nature. When antisense genes have been introduced into the cells and are stably maintained therein, the cells themselves (if transcription of the antisense gene takes place) will be able to produce in vivo antisense RNA. The resulting effects due to the antisense inhibition are then more lasting in nature than the inhibition obtainable when synthetic oligonucleotides are used.

Ellison et al. 1985, J. Biol. Chem. 260, 9085-9087 and Pestka et al. 1984, PNAS 81, 7525-7528 tested the effect of antisense β-galactosidase (lacZ) plasmids on the β-galactosidase production, using the $P_L$ promoter and a ts repressor mutant of phage lambda. At 42°C in medium containing IPTG (isopropyl-β-D-thiogalactopyranoside) the presence of plasmids containing antisense lacZ led to respectively 80% and 98% reduction of the β-galactosidase level in the E. coli cells. β-galactosidase, lactose permease and transacetylase are formed from the same polycistronic mRNA. When the antisense β-galactosidase RNA was formed, the lactose permease activity decreased by 80% and the thiogalactosidase transacetylase activity by 55%. The synthesis of the alkaline phosphatase formed from another mRNA template was, however, not inhibited.

In vivo antisense regulation also occurs with the E. coli genes coding for the lipoprotein (lpp) and the outer membrane proteins ompC and ompF by means of IPTG inducible lpp, ompC or ompF expression vectors (Coleman et al. 1984, Cell 37, 429-436).

Antisense RNA can be used to block the expression of cellular genes. Further, injurious viral genes penetrating into the cells from the exterior can be made ineffective by a similar regulator mechanism. In higher vertebrates such injurious viruses can be made ineffective by means of the immune system: as a reaction to the presence of body-foreign macromolecules proteins (antibodies) are formed having a high specific affinity against the undesirable molecules. The organism is immune to the injurious virus as long as the corresponding antibodies are present in the blood; the antibodies protect the organism against further infection. The antisense RNA technique, too, can be used as pathogenic "immune system"; then the high-specific molecules causing immunity are no antibodies (proteins) but antisense RNA molecules. As a model system for such an antisense "immune system" Coleman et al. 1985, Nature 315, 601-603, introduced antisense phage SP DNA into E. coli in order to prevent infection with this coliphage. Plasmids with antisense phage coat protein DNA and/or antisense phage replicase were transformed to E. coli. When much antisense RNA was formed via IPTG induction, no lysis of bacterial cells took place after phage infection. More than 90% inhibition of plaque-forming occurred. Experiments with $^3$H leucine showed that the synthesis of other proteins was not influenced. It was found later that an antisense phage SP protein A plasmid in E. coli provides an even stronger, almost complete resistance to phage SP infection. The antisense "immune system" can efficiently inhibit the multiplication of a specific virus.

## 3. Antisense RNA in eukaryotes

Besides as a template for protein synthesis, RNA is also naturally used in eukaryotes in regulation processes. Intramolecular base pairing in an eukaryotic mRNA may lead to the formation of a secondary structure whereby the translation of the transcript is inhibited. In the antisense inhibition mechanism, however, there is intermolecular base pairing. mRNA has often been associated with proteins both in the cytoplasm and in the nucleus. When considering possible antisense RNA regulation in eukaryotes, it should be realized that besides the mRNA in question the proteins associated therewith may also play an important role. mRNA splicing is an example of a natural process in which double-stranded RNA hybridization occurs. snRNAs (small nuclear RNAs) associated with small ribonuclear protein particles play a great part therein.

Certain RNA species isolated from eukaryotes may influence the translation in vitro. In muscle tissue from chicken's embryos tcRNAs (translation control RNAs) are present which inhibit the translation of the myosin heavy chain (MHC) mRNA. Since the 5' end of the MHC mRNA is highly complementary to the 3' end of a tcRNA being 102 nucleotides in length, it is assumed that this tcRNA is a natural antisense RNA of the MHC mRNA.

From chicken's embryo muscle tissue there have also been isolated other short transcripts which are complexed with proteins. These transcripts are different from the above discussed snRNAs and tcRNAs; they are strong inhibitors of the in vitro translation initiation.

It is possible that eukaryotic antisense transcripts consist of small RNAs which are perhaps complexed with proteins. From this point of view, it is interesting that many small transcripts are present in eukaryotes, the functions of which are not known. Examples thereof are the small nuclear RNAs which are transcribed into the 5' flanking region of the mouse gene for dihydrofolate reductase (DHFR). Synthesis of these small RNAs and the DHFR mRNA takes place in the opposite direction whereby they are partially complementary to each other. The functions of these small RNAs are still unknown. On the mouse chromosome a region is further present in which transcription of two mRNAs takes place in the opposite direction, so that the 3' untranslated ends overlap 133 bp. The functions of these transcripts are not known.

In the Drosophila genome, too, overlapping transcription units occur on opposite DNA strands. Here 88 bp overlap takes place between the genomic region coding for the 3' end of the dopa decarboxylase mRNA and the 3' end of the mRNA having an unknown function.

The cucumber mosaic virus (CMV) may also have an antisense regulator system. The genome of CMV consists of single- stranded RNA. In some CMV RNA isolations there is found, in addition to the RNA population generally present in CMV, a small single-stranded RNA: CMV satellite RNA. This satellite RNA requires CMV functions for its replication. In the CMV Q strain this satellite binds RNA in vitro to the CMV coat protein gene. Short complementary sequences of 23 nucleotides proved to be present, so that the satellite RNA may possibly wind RNA around the coat protein. It is plausible that this binding of the satellite RNA to the coat protein gene may regulate the synthesis of the coat protein. In antisense RNA regulation, antisense RNA : mRNA hybridization takes place, so that double-stranded RNA is formed. In RNA extracts of Xenopus oocytes dsRNA is actually present; the translation of this dsRNA proceeds badly, both in vitro and in vivo in injected oocytes.

Although not yet proved, there are therefore indications making it plausible that antisense RNA is a natural regulator mechanism not only in prokaryotes but also in eukaryotes.

Inhibition of the expression of endogenous genes with antisense RNA or those experimentally introduced into the cell is also possible with eukaryotes. For this purpose, in vitro synthesized antisense RNA or synthetic complementary oligonucleotides can be introduced into the cell. In addition, transcription of antisense genes introduced into the cell can be used for in vivo antisense RNA production.

In an in vitro translation system the translation is inhibited both after the formation of an mRNA : cDNA hybrid and after the formation of an mRNA : RNA hybrid.

If on behalf of certain biological systems no techniques are available for stable transformation of (antisense) genes, or if no efficient expression vectors are known, in vitro synthesized antisense RNA can be directly introduced into the individual cells via microinjection.

Melton 1985, PNAS 82, 144-148 examined the effect of the presence of antisense β-globin RNA on the formation of β-globin in Xenopus oocytes. Xenopus β-globin mRNA and antisense β-globin RNA were both synthesized in vitro by means of the bacteriophage SP 6 RNA polymerase system. Microinjection of globin mRNA into the cell cytoplasm of oocytes which normally form no globin led to synthesis of globin. When first antisense and 5 hours later sense globin RNA was injected, or when coinjection of these two transcripts took place, the synthesis of the globin was fully inhibited (antisense : sense ratio of 50 : 1). However, it proved to be difficult to obtain complete inhibition of an mRNA which was already present in the cell. When first sense and 5 hours later antisense globin RNA was injected into the cells, only a 10-fold inhibition of the globin synthesis occurred. This observation suggests that antisense globin RNA inhibits the globin translation, but that the extent of inhibition decreases after the mRNA has got into the polysomes. The inhibition was very specific: the translation of non-related transcripts was not influenced. Also the expression of the thymidine kinase (TK) mRNA injected into Xenopus oocytes and of the injected chloramphenicol acetyltransferase (CAT) gene can be inhibited with antisense TK RNA and antisense CAT RNA, respectively.

Besides heterologous genes, endogenous genes can also be inhibited with the antisense method by injecting antisense RNA into the cells. The Krüppel (Kr) gene has a very important function in the regulation of the segmentation of Drosophila embryos. Microinjection of Kr mRNA had no effect on the phenotype of the embryos. Microinjection of antisense Kr RNA gave a weak to strong (lethal) phenotypic response, depending on the amount of antisense RNA administered (Rosenberg et al. 1985, Nature 313, 703-706).

In the hitherto discussed experimental antisense inhibition in eukaryotes use was made of antisense transcripts which hybridized with a (nearly) complete mRNA. The antisense transcript, however, need not hybridize with the complete mRNA in order to inhibit the expression of the gene. Also short complementary RNA fragments or oligonucleotides can inhibit the protein synthesis. Inhibition of the gene expression by means of synthetic oligonucleotides has three advantages. First of all, it is easier to obtain a high molar concentration of a specific sequence. In addition, no clone of the gene in reverse orientation is required; a short piece of DNA sequence is already sufficient. Finally, during transcription long sequences beginning at the 5' end may fold in a secondary structure whereby they are no longer useful for hybridization with the complementary transcript. With short oligonucleotides this problem occurs to a far less extent or does not occur at all.

Besides in vitro, also in vivo hybridization of mRNA translation (HART) has taken place with short synthetic oligonucleotides. In Xenopus oocytes the expression of the heterologous interleukin 2 and 3 genes can be inhibited by more than 95% by means of coinjection of these genes with 18-23-mer synthetic complementary oligonucleotides. Microinjection into Xenopus blastomers of oligonucleotides which are complementary to the gap junction mRNA resulted in a Xenopus phenotype corresponding to that of gap junction deletion mutants. In human cells infected with human T cell lymphotropic virus type III or with the Herpes Simplex Virus type 1, oligonucleotides can be used to inhibit the virus replication and/or the expression of viral genes. Probably as a result of the time-bound degradation of the 20-mer oligonucleotides daily addition of the oligonucleotides after infection inhibited more effectively than a single, in total equally large dose. The Rous Sarcoma Virus production in chicken embryo fibroblast and the synthesis of the vesicular stomatitis virus in infected mouse L cells can be inhibited with oligonucleotides and oligonucleosides, respectively (nucleosides are resistant to nuclease hydrolysis and can penetrate into cultivated mammalian cells).

Although direct microinjection into cells of antisense RNA or of complementary oligonucleotides renders it possible to modify the amount of antisense RNA during the course of the experiment, antisense RNA would have to be injected again and again in case of a growing organism, because the antisense RNA becomes diluted or degrades in the course of time. Also, many cells cannot be injected, because they are too small or too difficult of access. Cell transformation due to the presence of a high nucleotide concentration in the culture medium is another possible approach. The antisense inhibition method, however, may have a wider range of application, if cells can be stimulated to produce their own antisense RNA. In the production of sufficient antisense transcript by means of an antisense gene introduced into the cell an effective promoter is the first requisite. Because genes which are essential to the normal growth and development of the cell cannot be inactivated permanently, inducible promoters are also desirable, so that the antisense RNA synthesis can be controlled.

Experiments with the thymidine kinase gene showed that an artificial antisense gene can be used to regulate the expression of a specific gene in eukaryotic cells. Introduction of plasmids with a sense TK gene into TK negative mouse cells leads to TK enzyme activity in the cells. Both coinjection, calcium phosphate coprecipitation and cotransfection (in TK negative cells) of a 100-fold excess of antisense TK genes relative to sense TK genes, led to a TK enzyme activity lower by 90-95% than was obtained after introduction of only sense TK genes. Autoradiography of the cells that still synthesized TK showed a very strongly reduced $^3$H thymidine incorporation. After removal of the promoter with the antisense TK gene and after coinjection with a sense TK gene no inhibition of the TK enzyme activity was found anymore. As anticipated, a stronger promoter for the antisense TK gene led to a stronger inhibition of the TK enzyme activity. In mouse cells the chloramphenicol acetyl transferase (CAT) and the lacZ gene expression were also inhibited after coinjection of plasmids containing sense and antisense CAT gene (Izant and Weintraub 1985, Science 229, 345-352) and cotransfection of plasmids containing sense and antisense lacZ gene (Rubinstein et al. 1984, C.R. Acad.Sci. 299, 271-274), respectively. Antisense inhibition of the c-fos protein in mouse fibroblasts has also taken place. The c-fos gene (cellular homologue of the Finkel Biskis Jinkins osteosarcoma virus transforming gene) plays an important role in the cell division of mammalian cells. Mouse fibroblasts were transformed with expression vectors containing sense and antisense c-fos gene. The production of the sense and antisense c-fos transcripts was regulated by the dexamethasone inducible mouse mammary tumor virus (MMTV) promoter. The presence of dexamethasone resulted in a 5- to 600-fold increase in the plasmid-encoded c-fos transcripts. Dexamethasone-induced production of the antisense c-fos RNA led to 90-96% reduction in the number of transformants. The process is reversible, since the inhibition disappeared, as soon as the dexamethasone had been removed (Holt et al. 1986, PNAS 83, 4794-4798).

Besides in animal cells, antisense inhibition has also been successfully applied in plant cells (Ecker and Davis 1986, PNAS 83, 5372-5376). For this purpose, plasmids were constructed in which the bacterial CAT gene was coupled in sense or antisense orientation to a number of different plant gene promoters, namely the promoter of the nopaline synthase gene (pNOS), the promoter of the Cauliflower Mosaic Virus 35S RNA gene (pCaMV) or the promoter of the phenylalanine ammonia lyase gene of the carrot (pPAL). Different ratios of sense and antisense CAT gene-containing plasmids were introduced into protoplasts of the carrot via electroporation. At a ratio of sense : antisense CAT gene-containing plasmids of 1 : 100 more than 95% reduction of the CAT enzyme activity occurred. The degree of inhibition of CAT enzyme activity correlated with the strength of the promoter on the antisense plasmid and increased if the antisense transcript contained a poly(A) signal.

In the hitherto discussed examples of antisense inhibition in eukaryotes the sense genes to be inhibited have been introduced experimentally into the cells via microinjection, transfection or calcium phosphate coprecipitation. Besides the expression of such artificial genes, the expression of genes endogenously present in the cell can also be inhibited with the antisense method by introducing antisense genes into the cell. That endogenous cellular genes in eukaryotes are also sensitive to antisense inhibition is shown by, inter alia, the antisense inhibition of the thymidine kinase gene in TK positive mouse L cells, the Drosophila heat shock protein 26 gene in Drosophila tissue culture cells, the mouse θ-actin gene in mouse L cells, the Dictyostelium discoidin 1 gene in Dictyostelium discoideum and the yeast iso-1-cytochrome c gene in Saccharomyces cerevisiae.

Rothstein et al. 1987, PNAS 84, 8439-8443 describes a transformation of tobacco plants with a nopaline synthase gene (Nos) of the bacterium Agrobacterium tumafaciens, followed by a superinfection with an antisense Nos gene under control of the strong CaMV 35S promoter. A reduced Nos enzyme activity was found in the resulting double transformates.

In a research conducted by Yokoyama and Imamoto 1987, PNAS 84, 7363-7367 a plasmid containing the antisense human MYC proto-oncogene under control of a Simian Virus (SV) 40 promoter was introduced into the human promyelocytic leukemia cell line HL-60 by protoplast fusion. The constitutive production of MYC protein could be reduced by 70%. The MYC expression proved to be inhibited not only on the level of translation, but also on the level of transcription.

Besides endogenous cellular genes, injurious (viral) genes penetrating the cells from the outside can also be made ineffective by means of the antisense technique.

One research was concerned with the polyoma virus Py (Amini et al. 1986, Mol. Cell. Biol. 6, 2305-2316) causing tumors. Use was made of rat cells which had been previously infected with the Py virus. Plasmids with an antisense Py c-src gene (cellular homologue of the sarcoma virus transforming gene) behind the inducible metallothionein promoter were transformed to these cells. $Cd^{2+}$ induction of this antisense gene led to 80-90% decrease of the Py-coded c-src protein synthesis. This decrease was dependent on the time and on the $Cd^{2+}$ concentration. The cells capable of expressing the antisense gene still caused tumor growth after injection into three weeks old Fisher rats. Although the cells had not been treated with $Cd^{2+}$, the tumor growth was clearly less than in the rats injected with the Py-containing parent cells (without antisense c-src gene). Probably, the subcutaneous level of heavy metals in these animals is high enough, anyway, to cause partial induction of the metallothionein promoter.

## Description of the invention

What will now be described for the first time is a use of antisense DNA in plants and plant cells in which the expression of a gene occurring in the plants or plant cells (also referred to hereinbelow as the host) is influenced, both quantitatively and qualitatively, by incorporating into the host recombinant DNA containing the relevant gene, or a gene sufficiently homologous thereto, in antisense orientation.

The present invention therefore relates to plant cells and plants provided through genetic engineering, optionally of an ancestor, with recombinant DNA comprising a promoter active in the host and a gene controlled by said promoter, said gene introduced having such a nucleotide sequence that on transcription an antisense RNA molecule is formed capable of forming a duplex with RNA molecules formed on transcription of a gene occurring in the DNA of the host.

More in particular, the invention relates to plant cells and plants provided through genetic engineering, optionally of an ancestor, with a recombinant DNA comprising a promoter active in the host and a gene controlled by said promoter, said gene introduced having such a nucleotide sequence that on transcription an antisense RNA molecule is formed capable of forming a duplex with RNA molecules formed on transcription of a gene of a secondary metabolic pathway occurring in the DNA of the host.

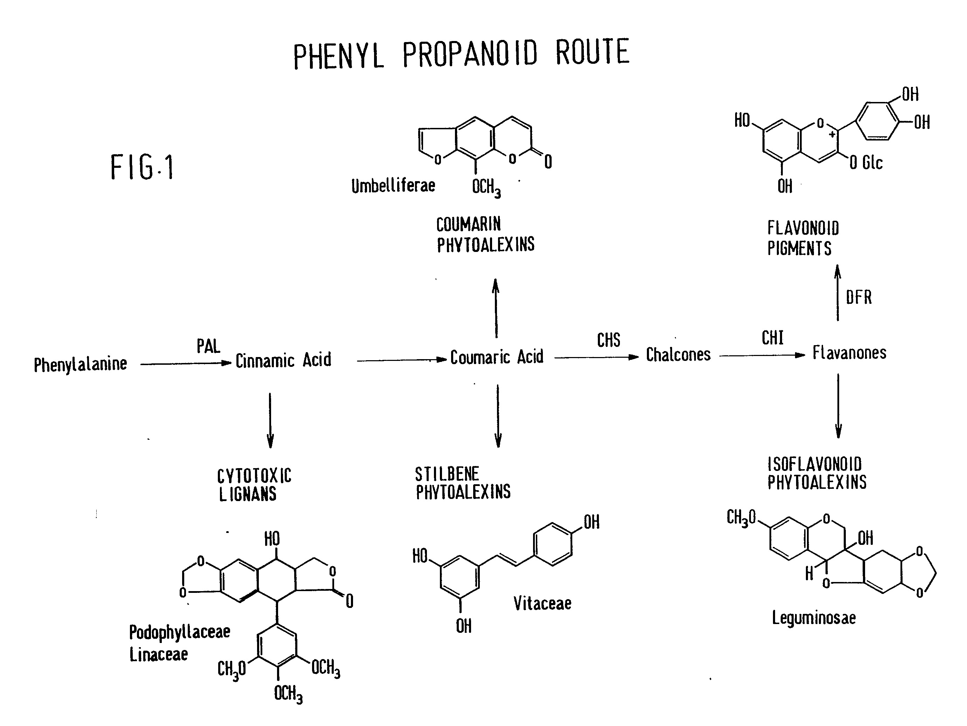

A preferred embodiment of the invention relates to plant genes of the phenyl-propanoid pathway (see Fig. 1), in particular the genes coding for one of the enzymes chalcone synthase (CHS), chalcone flavanone isomerase (CHI) and dihydroflavonol reductase (DFR). Most preferred is the gene for the CHS enzyme, because of its favourable position in the phenyl-propanoid pathway. Then the gene introduced consists preferably of antisense CHS cDNA of Petunia, e.g., antisense CHS (gene A) cDNA of Petunia hybrida, variety Violet 30 (V30). Although this antisense gene originates from Petunia, it has been found that it also exerts influence on the expression of the CHS gene in a different plant species, i.e. tobacco. Consequently, even when using this antisense gene originating from Petunia, the transgenic plant cells and plants according to the invention are not limited to Petunia. If desired, however, transgenic plant cells and plants of other plant species can be formed using an antisense gene with a higher degree of homology with the target gene of the relevant plant species, such as the target gene itself in antisense orientation.

The recombinant DNA introduced should, anyway, comprise a promoter active in the contemplated host and ensuring the possibility of transcription of the antisense gene into the host. As a rule, a strong promoter will be preferred. The promoter may be a constitutive promoter. The promoter may also be a regulable or inducible promoter. Examples of suitable plant promoters are known to those skilled in the art. Eminently suited is, e.g., the Cauliflower Mosaic Virus (CaMV) 35S promoter. The Ca MV 35S promoter is subject to developmental regulation. If the ratio mRNA/antisense RNA changes dramatically during organ development due to regulatory phenomena, patterns of gene expression will arise which should lead to phenotype variation within the developing tissue. Known to those skilled in the art are various plant promoters capable of being regulated or induced by specific chemicals, specific types of radiation or exposure to light, or by temperature changes.

The recombinant DNA introduced will preferably also comprise a 3' flanking region having therein a poly-A addition signal. But this is not always necessary, because the antisense gene may already contain the required poly-A addition signal. An example of a suitable 3' flanking region is the 3' tail of the nopaline synthase gene (Nos).

The invention not only relates to the above described transgenic plants and plant cells but also to recombinant DNA which can be used to form such transgenic plants and plant cells and, anyway, comprises the plant promoter and the antisense gene controlled by said promoter, and if desired, the 3' flanking region.

The recombinant DNA according to the invention may form part of a recombinant DNA vector which comprises a vector part in addition to the recombinant DNA according to the invention. As those skilled in the art will know, the vector part determines the host systems for which the recombinant DNA vector is suitable. For many host systems suitable vectors have meanwhile been known. For cloning purposes, use will mostly be made of a vector capable of replication in microorganisms, in particular bacteria, such as E. coli. The known pUC plasmids are very suitable for this purpose. Microorganisms transformed with a recombinant DNA vector according to the invention are also comprised by the invention.

The transformation of plants and plant cells requires the use of a suitable plant vector. Several of them, too, are known to those skilled in the art. A preferred vector is the plant vector Bin 19, as described by Bevan 1984, Nucl.Acids. Res. 12, 8711-8721.

Since the effect of the introduction of recombinant DNA according to the invention into plants or plant cells also manifests itself in future generations (stability in future generations has been established by way of experiment), the invention also comprises the progeny of the genetically engineered plants or plant cells which contain the genetic information of the recombinant DNA according to the invention.

The invention can be used for different purposes. Without being exhaustive, mention is made of the use in plant improvement and the use in the production of chemical agents, such as cytostatic agents, and of fermentation products, such as beer.

In particular when an antisense gene is used which influences the expression of plant genes that play a role in the biosynthesis of flavonoids, i.e. a special class of secondary metabolites involved in flower pigmentation, an important field of application is flower colour improvement. The biosynthesis of flavonoids proceeds via a branch of the general phenyl propanoid pathway shown in Fig. 1. The most important enzymes of this pathway are shown in the Figure, in which PAL stands for phenylalanine-ammonia lyase, CHS stands for chalcone synthase, CHI stands for chalcone flavanone isomerase and DFR stands for dihydroflavonol reductase.

As will be apparent from the experimental part, it has been established that flower colour variations can indeed be obtained with the invention. In these experiments use has first been made of DNA coding for the enzyme CHS. The key enzyme in the branch for flavonoids synthesis is chalcone synthase (CHS) which condenses 3 molecules malonyl CoA and 1 molecule coumaroyl CoA to naringenin chalcone. Isomerisation by chalcone flavanone isomerase (CHI) leads to the corresponding flavanone. Further reduction and substitution lead to the flavonoid flower pigments.

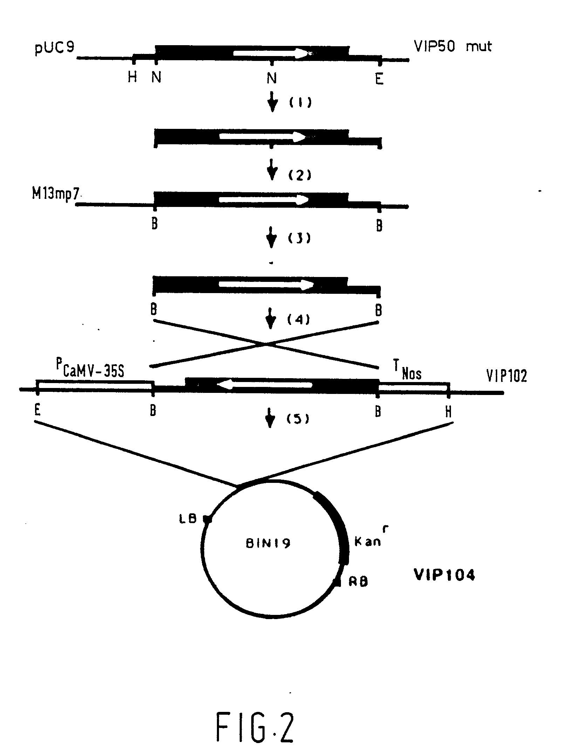

Like most of the plant genes CHS genes from Petunia hybrida form part of a multigene family. In the variety Violet 30 (V30) one CHS gene (gene A) is highly expressed (Koes et al. 1986, Nucl.Acids Res. 14, 5229-5239; Koes et al. 1987, Plant Mol. Biol. 10, 159-169). A second CHS gene (gene J) is expressed for about 10%. The CHS-A and CHS-J genes show about 86% sequence homology. It may therefore be expected that both will be inactivated by the same antisense CHS-A gene. cDNA corresponding to this active CHS gene A is fused in reverse orientation with the Cauliflower Mosaic Virus 35S promoter and the Nos 3' tail. This construct is then cloned into the plant vector Bin 19 (see Fig. 2).

This construct is introduced into petunia and tobacco by means of the leafdisc transformation method. Besides reduction in colour intensity, pattern formation was also observed for the first time, namely varying in independent transformants. Both in petunia and in tobacco there were also obtained completely white-flowering transformants in which the pigment synthesis is fully suppressed. The results convincingly show that gene expression can be modulated in a development specific manner or can be reduced nearly completely by means of antisense CHS DNA. The activity of the Petunia antisense CHS cDNA construct in a heterologous plant, such as tobacco, can be accounted for by the great homology between the Petunia and tobacco CHS genes. Up to now, all of the isolated CHS genes prove to possess a high degree of homology, and it may be expected that the Petunia CHS cDNA construct will be functional both inside and outside the solanaceae. Plants inside the species (potato) as well as outside the species (clover) have meanwhile been transformed so that a more general applicability of this CHS cDNA construct is shown. Genes for chalcone flavanone isomerase (CHI) and dihydroflavonol reductase (DFR) have meanwhile also been cloned from Petunia, and antisense constructs have been made. These two genes from the same biosynthesis pathway likewise offer perspectives within the scope of flower colour improvement. Manipulation of CHI expression, for instance, enables yellow chalcones to be accummulated in the limb resulting in new colour shades.

The proven possibility of obtaining a complete suppression of the pigment synthesis by means of the invention opens up perspectives for realizing pigment-free barley and hop in high yield, which is needed for the manufacture of beer. As described by Jende-Strid and Moller 1981, Carlsberg Res. Commun. 46, 53-64, pigments show coprecipitation with proteins, which gives rise to turbidities in the beer. Transformation of barley with, e.g., an antisense CHS gene is a potential alternative for existing methods for removing the pigment.

Fig. 1 shows that the phenyl propanoid (PP) pathway is of interest, because branches thereof lead to a whole range of biochemically related but functionally different compounds, such as flavonoids, phytoalexins and lignans. Manipulation with antisense genes from this pathway enables a specific control of the metabolic pathways to be realized.

By way of example, mention is made here of the lignan biosynthesis. Lignanes, such as podophyllotoxin, are recovered from free-living tropic plants and serve as a base for the preparation of cytostatics (e.g., teniposide and etoposide, derivatives of podophyllotoxin). The introduction of antisense CHS cDNA into cultured plant cells will enable the metabolic flow to be effectively directed from phenylalanine into the direction of the lignans, resulting in an increased yield. The use of antisense DNA may also be fruitful in controlling other secondary metabolic pathways.

Description of the drawings and experimental part

Fig. 1 is a schematic diagram of the phenylpropanoid pathway, a secondary metabolic pathway occurring in higher plants, branches of which lead to a range of biochemically related but functionally different compounds, such as flavonoids, phytoalexins, lignins and lignans. The abbreviations used are explained in the text.

Fig. 2 is a schematic diagram of the construction of a recombinant DNA vector according to the invention, which contains an antisense CHS gene. As a donor of the CHS sequence the cDNA clone VIP50 mut of the Petunia V30 CHS gene A was used with an NcoI site introduced in vitro with the initiation codon. This clone is described by Koes et al. 1986, Nucl. Acids Res. 14, 5229-5239. The VIP 50 mut was partially cut with NcoI and completely cut with EcoRI. The sticky ends were converted with the Klenow fragment of DNA polymerase I and dNTPs to blunt ends, after which the resulting fragment was cloned into the HincII site of phage M13mp7. The sequence coding for CHS was isolated as a BamHI fragment, which fragment was then cloned into the BamHI site of the plasmid pROKI (Baulcombe et al. 1986, Nature 321, 446-449), which site is located between the CaMV 35S promoter and a nopaline synthesis 3' flanking region containing the poly-A addition signal. The clone with the sequence coding for CHS in reverse orientation behind the CaMV 35A promoter (VIP102) was selected out for isolation of the EcoRI-HindIII fragment which was then cloned into the polylinker site of the binary vector BIN19, as described by Bevan 1984, Nucl.Acids Res. 12, 8711-8721. There was thus obtained the recombinant DNA vector VIP104 according to the invention.

The abbreviations used in the Figure for restriction sites are: E for EcoRI; B for BamHI; N for NcoI; and H for HindIII.

The recombinant DNA vector VIP104 was used to transform Petunia VR hybrids and Nicotiana tabacum SR1 plants. For this transformation VIP104 was mobilized to Agrobacterium tumefaciens 4404 by means of standard triparental pairing methods (see Ditta et al. 1980, PNAS 77, 7347-7351), after which the transformation of the plants was carried out according to the leafdisc transformation method (Horsch et al. 1985, Science 227, 1229-1231). The runner induction medium contains 250 ug/ml kanamycin for selection of cells expressing the neomycin phosphotransferase (NPT) gene present in the T-DNA region of BIN19. For each plant the transformation was confirmed by Southern blot analysis of genomic DNA. Total DNA was isolated from each transformant in the manner described by Koes et al. 1987, Plant Mol. Biol. 10, 159-169. An amount of DNA of 5 ug was cut with HindIII, after which the fragments were separated by electrophorese on a 1% agarose gel and transferred to a Genescreen Plus membrane (N.E.N. Research Prod.). The blot was analyzed with $^{32}$P-labeled NPT DNA or $^{32}$P-labeled CHS cDNA as a probe. Because treatment with HindIII would have to lead to unique fragments for each NPT copy integrated into the genome, the number of bands hybridizing with NPT was used as a measure of the copy number.

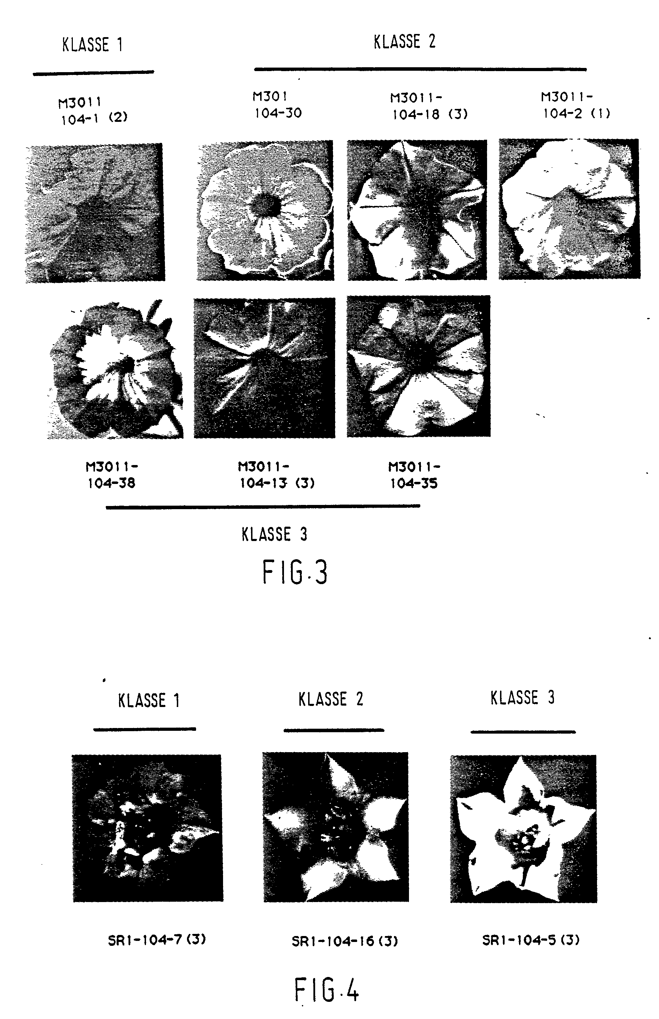

Fig. 3 shows a number of different transformants of Petunia VR hybrid. For some transformants the copy number mentioned is indicated between brackets.

The flowers of transgenic petunia plants were subdivided into 3 different classes of pigmentation type. Twelve out of the 25 transformants had flowers that could-not be distinguished from wild-type VR hybrid flowers (class 1, red coloured corolla, tube and anthers). Eight independent transformants showed a decreased flower pigmentation, which often resulted in alternating coloured and colourless (white) sectors, while in these transformants the tube was coloured (class 2). In five transformants (class 3) the colour pattern was fundamentally different from that in class 2 through an inhibition of the flower pigmentation from the tube and from there further to the outside into the corolla tissue, resulting in white flowers with a coloured ring or completely white flowers. The pigmentation of the anthers did not appear to be attacked in any of the transgenic plants.

An analysis of extracts from white flower tissue with thin-layer chromatography showed that flavonoids were absent. It further turned out that the blocking of the pigment biosynthesis could be avoided by adding naringenin chalcone (the normal product of CHS activity) to white corolla tissue. This addition led to colorization, from which it could be concluded that enzymes active in the phenylpropanoid pathway after CHS are present and active in these transgenic plants.

Fig. 4 shows that also in the transformation of Nicotiana tabacum $SR_1$ plants there are formed 3 different phenotypes. Of the 40 transformants, 36 had flowers that could not be distinguished from wild-type tobacco flowers (class 1). Three plants had flowers having a pigmentation divided into sectors (class 2) and one plant had completely white flowers (class 3).

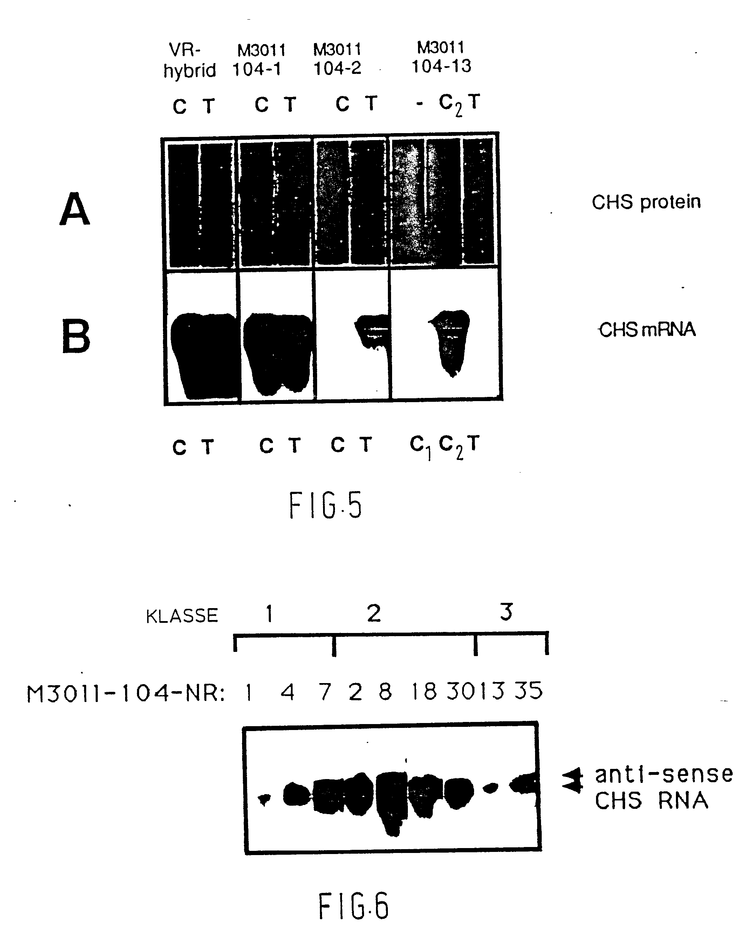

Fig. 5 shows in part A a Western blot and in part B a Northern blot analysis of petunia transformants of each class. Of each transformant, 5 to 7 still closed buds in their full length were collected for the analysis. Tube tissue and corolla tissue were separated and frozen in liquid nitrogen. Corolla tissue of transformant M3011/104/13 was separated into colourless ($C_1$) and coloured ($C_2$) parts.

The Western blots (A) were made in the manner described by Mol et al. 1983, Mol.Gen.Genet. 192, 424-429. For each sample use was made of equal amounts of protein extracted from either tube tissue (T) or corolla tissue (C). CHS protein was immunologically detected with rabbit CHS antiserum and alkaline phosphatase which conjugated with goat antirabbit IgG (of Tunen and Mol 1987, Arch. Biochem. Biophys. 257, 85-91).

The Northern blots (B) were made on Genescreen Plus Membrane from equal amounts (2 ug) of total RNA extracted from either tube tissue (T) or corolla tissue (C). CHS mRNA was detected with $^{32}$P-labeled antisense CHS RNA, which was obtained by transcription with T7 polymerase from the pTZ18U vector (U.S. Bioch. Corp.) which contains the EcoRI-HindIII fragment of VIP50. Hybridization and washing of the blot were carried out in accordance with the directions, but at 75° C instead of at 60° C.

The blot analyses showed a good correlation between the CHS protein amounts and the stable CHS mRNA levels in tube and corolla tissue, on the one hand, and the observed phenotype, on the other hand. Restriction analysis of genomic DNA of some transformants showed that in the endogenous CHS gene copies no changes had taken place. The above described changed pigmentation patterns were only observed in petunia plants which had been transformed with the antisense CHS gene. In normal or "sense" CHS gene constructs such deviating phenotypes were not observed (analysis of 20 transformed petunia plants). It is therefore improbable that these phenotypes should result from somaclonal variations, homologous recombinations or gene conversions.

Fig. 6 shows the results of a Northern analysis of the antisense CHS gene expression in leaves of the three classes of petunia VIP 104 transformants. With expression of the antisense CHS gene, both in transgenic petunia plants and in transgenic tobacco plants two different RNAs having lengths of respectively about 1300 bp and about 1100 bp are formed. These two different RNAs are attributed to the occurrence of polyadenylation on different sites, since in the antisense orientation of the CHS sequence 220 bp upstream of the Nopaline poly-A addition site a probably additional poly-A addition signal (AATAAT) proved to be present.

The procedure followed in this Northern analysis was similar to the above procedure described for Fig.

5B. Antisense CHS RNA was detected with $^{32}$P-labeled sense CHS RNA, which had been obtained by transcription with T7 polymerase from the pTZ19U vector (U.S. Bioch. Corp.), which contains the EcoRI-HindIII fragment of VIP50.

The results show that there is no correlation between the inserted number of copies of the antisense gene and the stable level of antisense CHS RNA in leaf tissue (compare in Fig. 6 results for 104-18 and 104-13). Apparently, adjacent sequences in the DNA have a quantitive effect on the gene expression. Furthermore, different independent transgenic plants having an equal stable level of antisense CHS RNA in leaf tissue show a difference in flower pigmentation (compare in Fig. 6 the results for 104-7 and 104-18). Some independent transgenic plants show a very low stable level of antisense CHS RNA in leaf tissue, while notwithstanding a distinct effect on the flower pigmentation is observed in those plants (see, e.g., 104-13 in Fig. 6).

The observations, among these in particular the occurrence of different classes of flower pigmentation, indicate that the integrated antisense CHS genes in the different transformants are expressed in different ways, apparently in conjunction with their position in the genome. The adjacent sequences seem to influence the gene expression also qualitatively (e.g., the timing of the gene expression and the spatial distribution of the gene expression).

Finally, it may be observed that the occurrence of an effect requires no antisense 5' leader sequence, in contrast to the situation with prokaryotes. Therefore, the inactivation of the gene expression does not seem to take place on the level of initiation of the translation, which presumption is also raised by the other observations.

## Claims

1. Plant cells and plants provided through genetic engineering optionally of an ancestor, with recombinant DNA comprising a promoter active in the host and a gene controlled by said promoter, said gene introduced having such a nucleotide sequence that on transcription an antisense RNA molecule is formed capable of forming a duplex with RNA molecules formed on transcription of a gene occurring in the DNA of the host.

2. Plant cells and plants provided through genetic engineering, optionally of an ancestor, with a recombinant DNA comprising a promoter active in the host and a gene controlled by said promoter, said gene introduced having such a nucleotide sequence that on transcription an antisense RNA molecule is formed capable of forming a duplex with RNA molecules formed on transcription of a gene of a secondary metabolic pathway occurring in the DNA of the host.

3. Plant cells and plants provided through genetic engineering, optionally of an ancestor, with a recombinant DNA comprising a promoter active in the host and a gene controlled by said promoter, said gene introduced having such a nucleotide sequence that on transcription an antisense RNA molecule is formed capable of forming a duplex with RNA molecules formed on transcription of a gene of the phenyl propanoid pathway occurring in the DNA of the host.

4. Plant cells and plants provided through genetic engineering, optionally of an ancestor, with a recombinant DNA comprising a promoter active in the host and a gene controlled by said promoter, said gene introduced having such a nucleotide sequence that on transcription an antisense RNA molecule is formed capable of forming a duplex with RNA molecules formed on transcription of a gene of the phenyl propanoid pathway occurring in the DNA of the host, coding for one of the enzymes . chalcone synthase (CHS), chalcone flavanone isomerase (CHI) and dihydroflavonol reductase (DFR).

5. Plant cells and plants provided through genetic engineering, optionally of an ancestor, with a recombinant DNA comprising a promoter active in the host and a gene controlled by said promoter, said gene introduced having such a nucleotide sequence that on transcription an antisense RNA molecule is formed capable of forming a duplex with RNA molecules formed on transcription of a gene of the phenyl propanoid pathway occurring in the DNA of the host, coding for the enzyme chalcone synthase (CHS).

6. Plant cells and plants, in which the gene introduced consists of antisense CHS cDNA of Petunia.

7. Plant cells and plants according to claim 5, in which the gene introduced consists of antisense CHS (gene A) cDNA of Petunia hybrida, variety Violet 30 (V30).

8. Plant cells and plants according to any of claims 1-7, in which the gene introduced is controlled by the Cauliflower Mosaic Virus (CaMV) 35S promoter.

9. Plant cells and plants according to any of claims 1-7, in which the gene introduced is controlled by a regulable or inducible plant promoter.

10. Plant cells and plants according to any of claims 1-9, in which the recombinant DNA introduced comprises a 3' flanking region having therein a poly A addition signal.

11. Plant cells and plants according to claim 10, in which the recombinant DNA introduced comprises the 3' tail of the nopaline synthase gene (Nos).

12. Recombinant DNA comprising a promoter active in plant cells and plants and a gene controlled by said promoter and having such a nucleotide sequence that on transcription an antisense RNA molecule is formed capable of forming a duplex with RNA molecules formed on transcription of a plant gene.

13. Recombinant DNA comprising a promoter active in plant cells and plants and a gene controlled by said promoter and having such a nucleotide sequence that on transcription an antisense RNA molecule is formed capable of forming a duplex with RNA molecules formed on transcription of a plant gene of a secondary metabolic pathway.

14. Recombinant DNA comprising a promoter active in plant cells and plants and a gene controlled by said promoter and having such a nucleotide sequence that on transcription an antisense RNA molecule is formed capable of forming a duplex with RNA molecules formed on transcription of a plant gene of the phenyl propanoid pathway.

15. Recombinant DNA comprising a promoter active in plant cells and plants and a gene controlled by said promoter and having such a nucleotide sequence that on transcription an antisense RNA molecule is formed capable of forming a duplex with RNA molecules formed on transcription of a plant gene of the phenyl propanoid pathway, coding for one of the enzymes chalcone synthase (CHS),chalcone flavanone isomerase (CHI) and dihydroflavonol reductase (DFR).

16. Recombinant DNA comprising a promoter active in plant cells and plants and a gene controlled by said promoter and having such a nucleotide sequence that on transcirption an antisense RNA molecule is formed capable of forming a duplex with RNA molecules formed on transciption of a plant gene of the phenyl propanoid pathway, coding for the enzyme chalcone synthase (CHS).

17. Recombinant DNA according to claim 16, in which the gene controlled by the plant promoter consists of antisense CHS cDNA of Patunia.

18. Recombinant DNA according to claim 16 in which the gene controlled by the plant promoter consists of antisense CHS (gene A) cDNA of Petunia hybrida, variety Violet 30 (V30).

19. Recombinant DNA according to any of claims 12-18, in which the gene is controlled by the Cauliflower Mosaic Virus (CaMV) 35S promoter.

20. Recombinant DNA according to any of claims 12-18, in which the gene is controlled by a regulable or inducible plant promoter.

21. Recombinant DNA according to any of claims 12-20, further comprising a 3' flanking region having therein a poly A addition signal.

22. Recombinant DNA according to claim 21 , comprising the 3' tail of the nopaline synthase gene (Nos).

23. Recombinant DNA vector comprising vector DNA and an insertion of recombinant DNA according to any of claims 12-22.

24. Recombinant DNA vector consisting of a plant vector and an insertion of recombinant DNA according to any of claims 12-22.

25. Recombinant DNA vector consisting of a vector suitable for cloning into a microorganism and an insertion of recombinant DNA according to any of claims 12-22.

26. Microorganisms transformed using a recombinant DNA vector according to claim 25.

# PHENYL PROPANOID ROUTE

## FIG.1

Umbelliferae

**COUMARIN PHYTOALEXINS**

**FLAVONOID PIGMENTS**

Phenylalanine → PAL → Cinnamic Acid → Coumaric Acid → CHS → Chalcones → CHI → Flavanones

DFR

**CYTOTOXIC LIGNANS**

**STILBENE PHYTOALEXINS**

**ISOFLAVONOID PHYTOALEXINS**

Podophyllaceae Linaceae

Vitaceae

Leguminosae

EP 0 335 451 A2

FIG·2

KLASSE 1

KLASSE 2

M3011
104-1 (2)

M301
104-30

M3011-
104-18 (3)

M3011-
104-2 (1)

M3011-
104-38

M3011-
104-13 (3)

M3011-
104-35

KLASSE 3

FIG·3

KLASSE 1

KLASSE 2

KLASSE 3

SR1-104-7 (3)

SR1-104-16 (3)

SR1-104-5 (3)

FIG·4

FIG.5

FIG.6